# EUROPEAN PATENT APPLICATION

(11) **EP 2 119 408 A1**
(43) Date of publication of application: **18.11.2009**
(21) Application number: 08156379.3
(22) Date of filing: 16.05.2008
(51) Int. Cl.: A61B 18/20

(54) **Photoablator**

(71) Applicant: TLT Medical Ltd, 4153 Reinach (CH)
(72) Inventor: The designation of the inventor has not yet been filed
(74) Representative: Vossius & Partner

(57) **Abstract**

A photoablator (1) for perforating a nail, having a laser source (2), optics (3) for focusing a laser beam emitted by the laser source (2) in a target plane, and beam positioning means (4) for positioning the laser beam in relation to the nail. The photoablator (1) further comprises a treatment area definition unit (13) for automatically defining an area of the nail to perforate by means of the laser beam. By means of the treatment area definition unit (13) the nail of a target finger or toe of a patient can be perforated respecting the specific conditions of the nail. Taking into account these conditions, the treatment area including a suitable treatment microhole pattern can be quickly defined by the treatment area definition unit (13), wherein according computing means can be used for this purpose.

## Description

### Technical Field

The present invention relates to a photoablator according to the preamble of independent claim 1. Such photoablators can be used for perforating a human nail to enhance the delivery of drugs or other agents to the nail plate, the nail bed or through the nail plate to other sites inside the body.

### Background Art

Human finger and/or toe nails are thick, hard, dense, and represent a barrier for drugs meant to penetrate down to the nail bed in a quantity capable of inducing significantly effective therapeutical action. The nail is primarily composed of hard keratin, which is highly disulfide-linked and is approximately 100-fold thicker than the stratum corneum of the skin being derived from epidermis. In order to deliver therapeutically significant amounts of drugs into and across the nail, the permeability of the nail plate can be enhanced by chemical, mechanical or by laser irradiation methods.

According to WO 02/11764 A2 nail diseases can be treated by forming with a laser one or more small holes into a nail of a patient and applying a therapeutic agent in a topical formulation to the nail. In order to secure a sufficient penetration of the therapeutic agent into the deeper layers of the nail and into the nail bed an array of holes in the nail plate is generated by means of pulses from a laser source subsequently filled with the topical formulation containing pharmaceutical active ingredients. However, the provision of holes into the nail can cause discomfort and pain to the patient if the nail perforation reaches the nail bed. Additionally, for providing the holes into the nail an area of treatment of the nail needs to be defined and monitored by an operator of an according device. This definition depends on various parameters, e.g., the surface structure of the nail, particularly including the contour and the topologic characteristics of the nail, the disease to be treated or the profile of the nail etc. Thus, performing this treatment area definition is a comparably difficult and time-consuming task such that the operator usually has to be comparably well-trained to perform this important task and the safety of the patient is at risk that the laser will perform, e.g., holes in the soft tissue surrounding the nail plate. Furthermore, there is a comparably high risk that a comparably inappropriate according definition is performed which can result in a decrease of the treatment efficacy and/or jeopardize the safety of the patient.

In EP 1 627 610 A1 a method and a device for treating nail diseases by forming with a laser an array of small partial holes or orifices into a nail are shown. Thereby, the partial holes or orifices penetrate 15% to 80% of the nail. A pharmaceutically active ingredient in a suitable topical composition is then applied to the nail and particularly inside the partial holes or orifices. Thus, significant amounts of ingredient can be delivered to the nail bed via the partial holes or orifices. However, for providing the partial holes or orifices into the nail an area of treatment of the nail needs to be defined and monitored by an operator of an according device. This definition depends on various parameters, e.g. the surface structure of the nail, particularly including the contour and the topologic characteristics of the nail, the disease to be treated or the profile of the nail, etc. Thus, performing this treatment area definition is a comparably difficult and time-consuming task such that the operator usually has to be comparably well-trained to perform this important task and the safety of the patient is at risk that the laser will perform, e.g., holes in the soft tissue surrounding the nail plate. Furthermore, there is a comparably high risk that a comparably inappropriate according definition is performed which can result in a decrease of the treatment efficacy and/or jeopardize the safety of the patient.

Therefore, there is a need for a device to perforate a nail allowing a comparably easy, fast, safe and efficient preferred treatment.

### Disclosure of the Invention

According to the invention this need is settled by a photoablator for perforating a nail as it is defined by the features of independent claim 1. Preferred embodiments are subject of the dependent claims. In particular, the invention deals with a photoablator comprising a treatment area definition unit for automatically defining an area of the nail to be perforated by means of the laser beam.

In this context, the term "photoablation" and derivations thereof as used herein refer to the vaporization of water in nail tissue and subsequent ejection of nail tissues induced by pulsed laser irradiation of a selected wavelength, power and pulse duration according to the thermal, mechanical and spectral characteristics of the nail of interest, which may be a finger or toe human nail. The deposited electromagnetic energy is almost entirely transformed into mechanical energy and the illuminated region is ejected in the form of debris escaping an orifice at high, often even supersonic velocities. As shown below in more detail, the deposited energy preferably can rapidly be removed from the nail together with the ejected debris therefore the irradiated nail is not heated locally and discomfort for the patient is minimized or inhibited.

The term "perforation" and derivations thereof as used herein relate to the creation of one or more holes, partial holes, or microholes in the nail, wherein the terms "partial hole" and "microhole" and derivations thereof relate to any small orifice or depression that penetrates 15% to 95% of the nail plate. Thereby, the lateral cross-sectional area can be of any shape, preferably a shape of a circle, oval, square, rectangle or another geometrical form or any intermediate shape of these including also irregular cross-sections. The diameter of the lateral cross-sectional area preferably is in the range of 1 micrometer to 1'000 micrometer, most preferably from 50 micrometer to 350 micrometer. Preferably, the axial cross-section area is of rectangular, trapezoid, parabolic, or triangular shape with regular or irregular shapes of the bottom.

The individual microholes of the perforation can be drilled in a raster scanning fashion where after one microhole is drilled the subsequent adjacent microhole to be drilled is next. Alternatively, the microholes of the perforation can be drilled in a random fashion where each microhole is drilled anywhere in the defined treatment area until all microholes of the perforation are being drilled. In this later fashion the heat in the surrounding area of each microhole is not carried to the next microhole increasing the temperature thus facilitating the dissipation of heat which is one of the sources of pain.

By means of the treatment area definition unit, the nail of a finger or toe of a patient can be perforated in a specific area without damaging the surrounding soft tissue. A suitable treatment microhole pattern can be quickly defined by the treatment area definition unit, wherein according computing means can be used for this purpose. For example, the treatment area definition unit can be embodied by configuring the laser beam to be retainable by a certain mask material protecting the soft tissue around the nail and/or the nail area not intended to be treated. Thereby, the mask material could reflect the laser light scattering in the instrument and/or absorb the laser light by having a given mass with sufficient heat capacity and heat conduction properties, so that the masking material does not change its temperature significantly as to be noticed by the patient. Examples of such masking materials could be plastically deformable metal foils, e.g. of aluminium or copper, or other easily deformable materials or cream-like formulations. In this configuration the array of spots illuminated by the laser beam is larger than the definition area to be treated, which is encompassed by the mask.

In addition to the beam-positioning means, the photoablator can also comprise an optical divider to create several laser beams from the original laser beam output for creating an array of microholes having a predetermined preferred pitch. The term "array of microholes" and derivations thereof as used herein relate to a set of partial holes generated in a regular pattern, e.g., in an orthogonal pattern where four neighbouring microholes form a square, or, e.g., a hexagonal pattern where one microhole has six neighbouring holes in identical distance. The density of microholes in an array is typically about 100 to 1000 orifices per square centimeter, preferably between about 350 to 450 orifices per square centimeter. The term "pitch" and derivations thereof as used herein relate to the distance between two adjacent microholes in the array. The pitch is typically is between about 0.1 and 5 millimeter, preferably between about 0.5 and 1 millimeter. The optical divider can be a diffractive optical element such as Dammann grating or beam splitters.

Preferably, the treatment area definition unit comprises area detection means. With such area detection means the conditions of the nail to be perforated can automatically be analyzed in a precise and fast manner. For example, the treatment area definition unit can be arranged to be capable of detecting the edge between the nail and its surrounding soft tissue via the area detection means and computing the treatment area and microhole pattern including a safety margin between the nail and the soft tissue. The treatment area definition unit can also automatically detect only a specific section of the nail, e.g., a diseased part of the nail. The nail can then be perforated by the laser beam corresponding to the performed definition of the treatment area. For example, the area detection means can be capable of detecting the edge between the nail and its surrounding soft tissue using, e.g., a visual element such as an optical sensor (e.g. a CCD or a video camera) coupled to a pattern recognition software. Such treatment area definition unit featuring the pattern recognition software can also be programmed to automatically detect a specific section of the entire nail, e.g., a diseased part of the nail.

In one preferred embodiment, the area detection means comprise an optical sensor. Thereby the optical sensor can be, e.g., be arranged for detecting a marked area of treatment of the nail, wherein this area is marked in advance with, e.g.,, a suitable pen to facilitate the automatic recognition process. For this purpose, the optical sensor can alternatively be, e.g., a fluorescence detector, wherein a fluorescence formulation can be applied to the nail prior to detection.

Further, the optical sensor can be a laser topography measurement sensor or a laser triangulation sensor. The measuring laser beam then, e.g., scans the complete nail and surrounding soft tissue and the treatment area is defined based on the detected edges between the nail and the soft tissue. Other optical sensors can be, e.g., interferometers, optical coherence homographs or line projection devices. Still further, the optical sensor can be either arranged such that various light sources illuminate the nail from different directions and the shadows on a provided image are analysed, or as a 3D-photo camera for providing a stereoscopic image.

In a further preferred embodiment, the area detection means comprise an acoustical sensor. With such an acoustic sensor the echo of an emitted sound can be evaluated to analyze the conditions of the nail to be perforated and the surrounding skin tissue. The acoustical sensor can, e.g., comprise a pressure transducer such as a piezo electric element or a microphone. The acoustical sensor can also be used to detect the sound generated by a pulse of light from a photoacoustic effect in the tissue, and such information can be used again to analyse the conditions of the tissue at that spot. For this purpose, a laser source, preferably the same laser source as used for the nail perforation, can be used.

Thereby, the acoustical sensor preferably comprises sound evaluating means. With such sound evaluating means changes in sound characteristics e.g. caused by single laser pulses on the tissue surface, can be detected and analysed. Such changes in sound characteristics can be changes in the acoustic impedance of the tissue, the frequency pattern or the amplitude of the sound wave. Since the sound characteristics of the skin and the nail surface are different from each other, the system recognizes when the first laser pulse of a pulse series hits skin tissue and can stop perforation in this spot. This method can be used for area definition and limitation of perforation to the nail plate by always analysing the first laser pulse during laser perforation of a micro hole array before sending the complete pulse series to one single spot. If the sound characteristics correspond to nail tissue, the perforation is continued at the same spot to create a partial microhole. If the sound characteristics do not correspond to nail tissue, the laser beam is positioned to another location to analyse the tissue for perforation again. Preferably, for such a method the first pulse of a pulse burst has a low energy so that its photoablation hole does not reach sensitive and blood-circulated layers of the skin. This real time area definition method can be optimized with specific scanning algorithms to achieve a fast laser treatment of the complete nail plate.

Preferably, the photoablator further comprises a drilling feed-back unit with means for detecting a property of the tissue being perforated by the laser beam of the photoablator wherein the drilling feed-back unit stops the laser beam when the tissue property has a predefined value. Since various tissue properties of the nail relate to the distance from the soft tissue of the nail bed, these tissue properties of the bottom of an individual microhole change with decreasing distance between the bottom and the nail bed. Thus, the drilling process can be stopped automatically, irrespective of the thickness of the nail, before the laser beam reaches the nail bed, which can be painful. For controlling or stopping the drilling process, the drilling feed-back unit advantageously can be operatively coupled to means for controlling the drilling process such as the laser source or the beam positioning means.

Considering that the changes in the nail thickness over the whole nail plate are usually smooth, a dedicated software can be used to, e.g., store and remember the number of pulses from a previously drilled microhole in one or some representative parts of the nail to speed up the overall process using a dedicated algorithm. Furthermore, with this dedicated software the characteristic of an individual microhole can be compared with the predetermined value such as, e.g., an acoustic waveform or a fluorescence at a given wavelength, and laser pulses illuminating the individual microhole can be stopped or deviated when the characteristic of the individual microhole match the predetermined value. Also, the accuracy of the final depth of the individual microholes can be improved by varying the laser energy as a function of the depth of the individual microholes to, for example, decreasing the laser energy of each pulse from the first pulse of each spot in the nail until the process is stopped. Thus, the drilling feed-back unit allows ensuring a comparably comfortable, fast, and efficient perforation of the nail.

In a preferred embodiment, the drilling feed-back unit comprises an acoustical sensor. Such an acoustical sensor can be made of a pressure transducer such as, e.g., a piezoelectric element or a microphone. By means of such an acoustical sensor, the characteristics of the microhole can be detected, such as changes in the acoustic impedance of the nail, the frequency pattern, or the amplitude of the sound wave for each photoablation step caused by each pulse, and the detected values can be used for controlling and/or stopping the driving process. Thus, such an acoustical sensor allows an efficient control of the drilling process.

Thereby, the acoustical sensor preferably comprises sound evaluating means. With such sound evaluating means a change of sound characteristics of the microhole, e.g., caused by the sound of single laser pulses, can be detected and analysed. Since the sound characteristics of the nail bed, the nail surface and lower layers of the nail usually differ from each other, certain specific changes of sound characteristics correspond to very short distances of the bottom of the microhole to the nail bed, so that the system can stop sending subsequent laser pulses to this spot in such a case. Thus, such sound evaluating means allow an efficient control of the drilling process.

In a further preferred embodiment, the drilling feed-back unit comprises an optical sensor. Such an optical sensor can have a measurement system like a spectrograph, a fluorescence detector, a laser triangulation device, an interferometer, an optical coherence tomograph, a line projector, or a laser scanning device. Alternatively, the optical sensor may have various light sources that illuminate the microholes, for example illuminate the microholes from outside the microholes, or a 3D-photo camera for providing a stereoscopic image, and means for analysing the shadow generated inside the microholes or the stereoscopic image. By means of such an optical sensor, characteristics of the microhole can be detected, analysed and used for controlling and/or stopping the drilling process.

Thereby, the optical sensor preferably comprises color evaluating means. With such an optical sensor a change of color of the bottom of the microhole can be detected and analysed. Since the color of the nail bed usually is different from the color of the nail, such a change of color can correspond to the immediate vicinity of the bottom of the microhole to the nail bed, in which situation the drilling process in this specific microhole is stopped. Thus, such color evaluating means allow an efficient control of the drilling process.

In a preferred embodiment, the photoablator comprises a debris capture with a pump, a coarse filter and a fine filter, for evacuating debris from the nail. The pump can be a vacuum pump to extract the debris from the nail or an overpressure pump for blowing the debris off the nail. The term "coarse filter" and derivations thereof as used herein relate to a mechanical filter being capable of retaining comparably large particles and could be realized by, e.g., glass wool or glass fibers. Such a filter entraps particles in the range of 1 micrometer up to 1 millimeter, preferably 30 micrometer to 500 micrometer. The term "fine filter" and derivations thereof as used herein relate to a component containing chemically active substances, such as, e.g., active coal particles, and a lower mesh membrane embedded in, e.g., glass wool or glass fiber or any other supporting porous structure. Alternatively, the fine filter could also be an electro filter or any comparable filter capable of fulfilling the same functionality, i.e. to filter odor producing particles and possible pathogens from escaping the photoablator. Indeed, such fine filter is meant to entrap molecules and particles in the range of 10 nanometer up to 100 micrometer, preferably 1 micrometer to 50 micrometer. Most conveniently, the coarse filter is placed before the fine filter in the sense of the air flow and debris stream coming from the nail. Thereby, the pump preferably is connected by means of plastic tubes with the coarse filter and the fine filter. In a preferred embodiment the coarse filter is a single-use component only used once per patient, therefore designed to be used for the laser treatment of 1 to 20 finger and/or toe nails, and the fine filter is a multiple use component designed for, e.g., 50 to 500 patients before its replacement or servicing. In another preferred embodiment, the coarse filter and the fine filter are incorporated in a single housing and designed for single-use for one patient, therefore again designed to be used for the laser treatment of 1 to 20 finger and/or toe nails.

In a further preferred embodiment, the photoablator comprises a debris capture with adsorption means for adsorbing debris to evacuate the debris from the nail. The term "adsorption" and derivations thereof as used herein relate to adsorption in the true sense of the word as well as adherence or attraction. Such adsorption means, e.g., being arranged as surfaces laying in the trajectory of the ejected photoablation particles, normally over the nail to be treated, allowing sufficient transmission for the laser beam to create appropriate microholes in the nail plate while adsorbing debris particles. Such surfaces may also be of specific geometrical configuration to entrap photoablation particles. The efficacy of such entrapping surfaces can be enhanced by a pump or other means generating an air stream which directs the particles to the entrapping surfaces. Preferably, such surfaces are coated with a chemically active substance to bind or neutralize odor generating molecules. Examples of such surfaces can be porous surfaces or surfaces with fine ribbed structures with active charcoal coating.

By removing the debris from the nail with a debris capture as described above, the optical components of the photoablator can be kept clean near the nail being treated during the whole photoablation process. Thus, contamination of the optics of the photoablator is avoided or reduced to retain a constant photoablation efficiency through the whole treatment which is otherwise compromised by a decrease in the transmission of the optics when dirty. The benefits of a debris capture are thus that pathogens, molecules and particles can be retained within the photoablator, allowing a reduction of odor produced by the photoablation of the nail, keeping debris away from the optics of the photoablator and protecting users and patients from pathogens.

Preferably, the photoablator further comprises a docking unit for fixing the nail in a predefined position. With such a docking unit a precise perforation of the nail by photoablation is efficiently possible. In one possible configuration the relative positioning of the nail with respect to the laser beam waist can be realized by resetting the nail against a mechanical stop. Thereby, a spring loaded clamp or a similar element can be used to compensate for the shape diversity in fingers and toes. Elements pushing the finger or toe against the mechanical stop can be elastic or visco-elastic foam patches, hinged clamping elements or combinations thereof. The docking unit preferably is designed so that it allows ergonomic and comfortable positioning of the finger/toe during laser treatment. Further, the docking unit can be designed in such a way that it prevents laser light to escape the photoablator into the free-space and constructed by materials and design features that reduce noise caused by the photoablation process. Thus, a convenient, precise, and safe application of the photoablator is possible.

The docking unit can also have a transparent window to keep the optical components of the nail laser photoablator clean during the photoablation while still allowing the treatment of the nail with the laser beam and the visualization of the nail by means of a monitoring video element or other optical sensors. To avoid cross contamination between patients, the docking unit preferably is arranged washable and sterilizable by common means or as a single-use device to be disposed after use in one patient. Such a single-use docking unit could also incorporate the single-use filter system as described above.

In a preferred embodiment, the photoablator has a treatment module comprising the laser source, the optics, the beam positioning means, and a gripping portion and a docking module comprising the docking unit, wherein the treatment module is arranged for being manually dislocatable by means of the gripping portion and wherein the docking module is arranged to accommodate the treatment module while the nail is fixed in the predefined position. The docking module can be arranged for single use of individual patients. Thereby, the docking module can be provided in different dimensions and shapes according to the fingernail or toenail to be treated such that the optimal fixation of fingers and toes of different sizes and forms is possible and the treatment can be adapted to the individual patient.

The docking module and/or the treatment module can preferably have a mechanical shutter or an optical diaphragm opening when the docking module accommodates the treatment module. This allows enhanced optical safety of the photoablator since the shutter or the diaphragm can be otherwise closed, e.g., when the photoablator is warming up. Thereby, the docking module is arranged in such a way that the optical path of the photoablation laser beam starting in the treatment module preferably finishes at the nail. In this way, the laser beam is always jacketed inside the treatment module and the docking module for enhanced patient and operator safety. The shutter can be located in any place inside the photoablator but most preferably at the exit of the docking module or the treatment module where the docking module accommodates the treatment module.

Furthermore, the treatment module can have a docking port which is connectable to the docking module. In particular, this docking port can be arranged at a housing of the treatment module. The docking port allows a safe connection between the treatment module and the docking module. The docking port can also assure correct positioning of the docking module in regards to the laser beam and its focal plane and therefore in regards to the beam positioning means, to the drilling feed-back unit and to the treatment area definition unit.

When an exchangeable docking module is used and a debris capture is implemented in the treatment module, the debris capture can also be connectable to the docking module to fulfill the removal function. The debris capture can also be incorporated into the single-use docking module. In such configuration the docking module can comprise a window of a transparent material allowing the laser beam to propagate without substantial loss of energy. In this way, an in-situ optical inspection by suitable means is possible, e.g., for the drilling feed-back unit or the treatment area definition unit.

In another preferred embodiment the drilling feed-back unit can be combined with the treatment area definition unit into one single physical entity or they can share single physical entities for performing specific functions. For example, the same acoustical sensor can be used on one hand to detect a sound property of the laser pulses striking the nail or other tissue for getting the drilling feed-back. On the other hand, the same acoustical sensor can be used for defining the treatment area by providing a first laser pulse in a given spot to discriminate if the tissue being hit by this first laser pulse is soft or hard depending on the changes in, e.g., the acoustic impedance of the nail or the amplitude of the sound wave. In this way, when the spot lies, e.g., on the soft tissue surrounding the nail the drilling process can be stopped and the beam positioning means return the laser beam to another area continuously defining the contour by means of appropriate software. Thus, in this configuration the treatment area definition is performed during the actual laser treatment. By stopping the laser and positioning the beam to the next spot whenever the acoustic sensor determines that the tissue being irradiated is soft, e.g. in the tissue surrounding the nail plate or in the nail bed, the safety of the photoablator is in this way substantially enhanced.

Summarizing the present invention, the photoablator employs a laser beam to create, by means of fast photoablation, an array of microholes of pre-determined pitch in human nails to enhance the permeation fluxes of a pharmaceutical topical formulation and its contained drug applied to the dorsal part of the laser-treated nail plate and filling the microholes. Prior to the laser treatment, various laser parameters may have to be determined. Included are: Pulse energy, pulse duration and frequency as well as the geometrical arrangement of the microhole array. Preferably, the laser beam delivers light having a wavelength of 2.94±0.2 micrometer, but it could also emit radiation at other wavelengths, where water has strong absorption bands.

A preferred laser source is a solid state diode pumped featuring a Q-switching device to shorten the pulses and increase laser repetition rates of light pulses. Laser diode pumping is more efficient in terms of energy conversion than flash lamp lasers and allows a much faster repetition rate. Preferably, one or more laser diodes are used to pump the laser gain media. Preferably, pulses having a temporal width of less than 250 microseconds are used, most preferably between 50 and 150 nanoseconds. The pulse repetition frequency of the laser source preferably is higher than 200 Hz, most preferably higher than 500 Hz provided that the width and energy of each pulse is capable to produce efficient photoablation. Under such conditions the total time to create the array of microholes in, e.g., a single large toenail is less than 1 minute and preferably less than 30 seconds. One of the benefits of achieving short period of treatment time by efficient diode pumped laser is that the comparably low amount of heat generated by the laser gain media in this period could be dissipated to the air by other elements of the photoablator such as the housing or mounting elements or a combination of both. In particular, in such a configuration the thermal energy stored temporarily in the device components can be dissipated before the next array of microholes is performed in another fingernail or toenail of the same patient. Therefore, in a preferred embodiment, the photoablator requires no active cooling means such as water or any other liquid circulation or a ventilator to dissipate further energy. However, in another embodiment, the running temperature of the laser could be controlled by means of a ventilator or by a thermoelectric temperature controller featuring, e.g., Peltier elements as described by A. Bruno et. al. in Anal. Chem. 63(23), p. 2689 (1991). Such active temperature controller would allow a comparably easy miniaturization of the photoablator while, at the same time, the beam laser characteristics are not degraded during the operation due to thermal fluctuations as e.g. due to thermal lensing.

Another critical parameter in clinical practice is the shortening of the whole treatment time for a procedure which is achieved in the present invention by using a laser operating at repetition rates above 20 Hz, preferably higher than above 200 Hz, most preferably higher than above 500 Hz. Repetition rates above 200 Hz allow the drilling of individual holes with multiple pulses in a very short period of time. Also, to achieve an overall short treatment time, other processes contributing to the overall duration of the treatment have to be fast such as, e.g., the positioning of the laser beam from one spot to another, the time devoted to define the area of treatment, and the time required to control to the last shot when drilling individual microholes. Therefore, in the preferred embodiment of this invention all processes contributing to the overall duration of the treatment are automated. In particular, by incorporating the definition of the area of the nail plate by means of the treatment area definition unit and, the drilling feed-baclc unit having, e.g., a feedback loop to stop the laser drilling of individual microholes before the nail is fully traversed. These improvements allow reducing the overall treatment duration of, e.g., a single large toenail to be treated in less than 1 minute.

Thus, the present invention results in a significant improvement over the prior art by addressing issues relevant to the patients and users of the photoablator without jeopardizing the efficacy of the treatment in question. Such relevant issues can include short treatment duration, small ergonomic design suitable for point-of-care treatments, sophisticated debris evacuation system which inhibits odor and pathogens generated by the photoablation escaping the nail photoablator into the air, acoustic isolation resulting in minimal amount of noise, definition of the treatment area and a means to stop the laser of drilling individual microholes before the whole nail is traversed to eliminate pain and prevent pathogens to reach the nail bed.

### Brief Description of the Drawings

The photoablator according to the invention is described in more detail hereinbefow by way of exemplary embodiments and with reference to the attached drawings, in which:
Fig. 1 shows a schematic view of a first embodiment of a photoablator according to the invention having two modules; and
Fig. 2 shows a schematic view of a second embodiment of a photoablator according to the invention having three modules.

### Mode(s) for Carrying Out the Invention

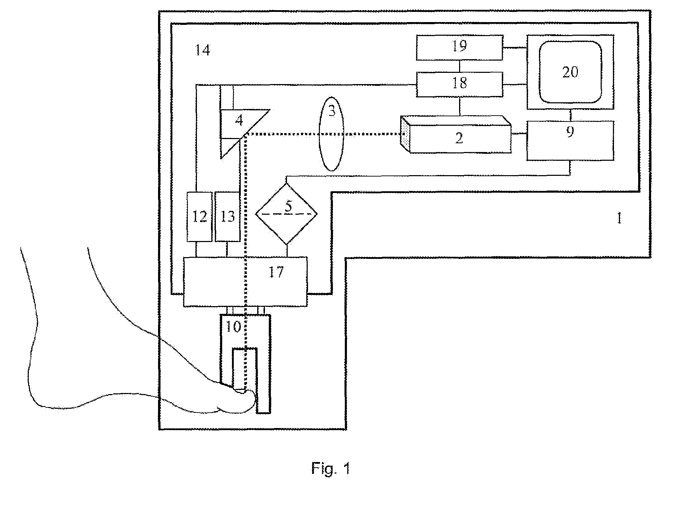

Fig. 1 shows a nail photoablator 1 comprising a docking module 10 and a treatment module 14. The treatment module 14 comprises a photoablator laser 2 as laser source, optics 3, a beam positioner 4 as beam positioning means, a debris capture 5, and a docking port 17 to attach the docking module 10. The docking module 10 is arranged to fixedly position the finger or toe nail to be treated. The debris capture system 5 can optionally comprise an easy to dispose filter cartridge as coarse filter to capture the large size debris particles and another high capacity filter element as fine filter intended to capture the remaining debris particles and gases to prevent pathogens and odor to escape the photoablator 1. The filtering system is arranged either to be removable or to be accessible for being serviced periodically. The debris capture system 5 comprises a vacuum pump to allow the optical components near the nail being treated to remain clean during the whole photoablation procedure.

The treatment module 14 of the photoablator 1 also comprises a main power supply unit 9, a user interface 20, a drilling feed-back unit 12 to control and stop the laser drilling of individual partial microholes before traversing the entire nail, and a treatment area definition unit 13 to define the area of laser treatment. Further, the treatment module 14 has computing means 18 running dedicated software 19 to control the entire photoablator and the therapy.

The treatment unit 14 of the photoablator 1 further comprises a comparably small-sized portable or ergonomic hand-held housing to contain all of its components mentioned above and below. The treatment module 14 can also have a visual monitor to observe the nail, follow the treatment in real-time, capture images of the nail at different points if required to, e.g., use pattern recognition methods for definition of the area of treatment. The hand-held ergonomic treatment module 14 including its interior components is arranged such that it has low weight and is comfortable to fit in the hand of the nail laser photoablator operator. Preferably, the length of the treatment module 14 is between 10 and 50 centimeter, the width of the treatment module 14 is between 10 and 30 centimeter and the height of the treatment module 14 is between 5 and 30 centimeter, as well as the total weight of the treatment module is in the range of 100 gram and 10 kilogram. The docking module 10 is arranged to be easily movable and placeable on, e.g., a table to handle finger nails or on the floor to treat, e.g., toenails.

A preferred gain media for the photoablator laser 2 is a solid media which is preferably selected from the group of Erbium or Holmium solid state lasers such as: Er:YAG, Er/Pr:YAG, Ho:YAG, Er/Cr:YSGG, Ho:YSGG,, Ho/Nd:YAG provided that they emit at wavelengths, where water has strong absorption bands and the wavelength is not otherwise harmful to the human body. Diode lasers, fiber lasers, or any other laser capable to photoablate the nail tissue and where the output wavelength is not otherwise harmfull to the human body can also be used in the photoablator.

The photoablator laser 2 has a pulse temporal width which is between 1 nanosecond and 1 millisecond, in particular, between 10 nanosecond and 300 microsecond and, most preferred, between 10 nanosecond and 150 nanosecond. The photoablator laser 2 also delivers an energy density of a laser beam between 1 millijoule per square centimeter and 100'000 joule per square centimeter, in particular, between 10 millijoule per square centimeter and 5 joule per square centimeter.

The optics 3 of the treatment module 14 are composed of one or several lenses in order to focus the laser beam in a target plane, where the nail is positioned, to an energy density required to generate a single pulse microhole of defined diameter and depth. The photoablator 1 preferably uses optics 3 focusing the laser beam to an energy density between 1 millijoule per square centimeter and 100'000 joule per square centimeter, in particular between 10 millijoule per square centimeter and 5 joule per square centimeter. The lenses of the optics 3 have a fixed configuration with a beam waist range in the focal plane of about 4 millimeter allowing accurate perforation also of curved nails without focus adjustment. Alternatively, an optical focusing element is incorporated into the optics 3 comprising at least one lens that can change its distance with respect to the dorsal surface of the nail to ensure that the waist of the focused laser beam strikes the nail on its surface or at the level of perforation inside the microhole to be generated.

The beam positioner 4 is either arranged by placing mirrors, e.g. one or more galvanometric deflectors, or using a two or three dimensional mechanical scanner or combinations thereof. The beam positioner 4 has two mirrors reflecting the laser beam into the required direction illuminating the treatment area at different angles and at different places. Alternatively, the beam positioner 4 is arranged with a mirror having a fixed 45°-angle reflecting the laser beam in an angle of 90° and being moveable in x- and y- direction to position the laser beam propagating in the z-direction on the target nail, so that it meets a plane of the nail always in the same angle. In addition, the beam positioner 4 can also be a combination of the above mentioned two mirror systems so that the beam is enabled to meet the nail surface always in a more or less perpendicular angle. Each microhole is drilled by at least two laser pulses in the same spot but, depending on the characteristics on the laser beam in terms of electromagnetic power, pulse duration and repetition rate (operating frequency) and on the thickness of the nail to be drilled, the number of pulses to drill a single microhole could be as high as 1'000 pulses.

The treatment area definition unit 13, the drilling feed-back unit 12, and the debris capture 5 are arranged as described above.

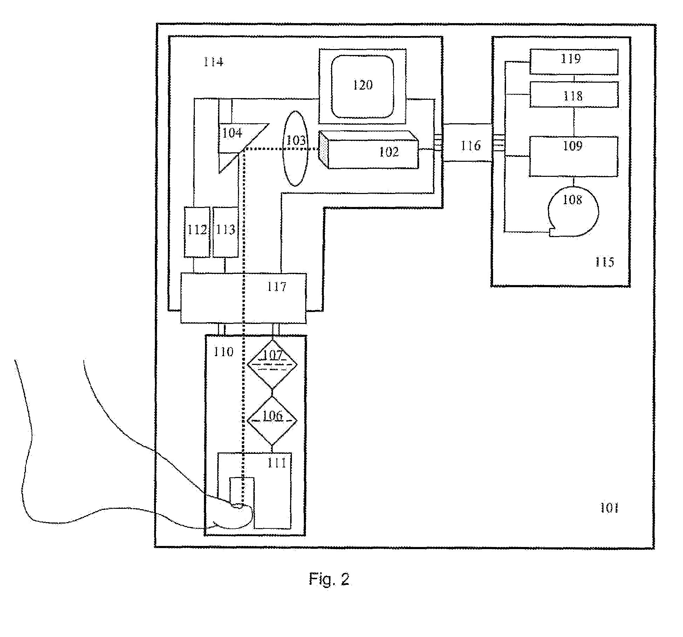

In Fig. 2 a photoablator 101 having a docking module 110, a treatment module 114, and a remote module 115 is shown. The treatment module 114 comprises a photoablator laser 102 as laser source, optics 103, a beam positioner 104 as beam positioning means, a docking port 117, a user interface 120, a drilling feed-back unit 112, and a treatment area definition unit 113. The docking module 110 comprises a docking unit 111, a coarse filter 106, and a fine filter 107. The remote module 115 comprises a main power supply unit 109, a computer controller 118 running a dedicated software 119, and an air pump 108 as a component of the debris capture. All of these components are arranged the same way and provide the same functionality as the corresponding components described above if not adversely described below.

The docking unit 111 is arranged to be easily disposable, wherein it is replaceably connected to the docking module 110 such that it can be easily removed from the docking module 110 and be disposed. Alternatively, also the docking module 110 is arranged to be easily disposable. The remote module 115 is attached to the treatment module 114 by means of a flexible connector bundle 116 comprising electrical cables and plastic tubes. The remote module 115 comprises the pump 108 of the debris capture wherein it is connected to the treatment module 114 and the docking module 110 via the plastic tubes. The computer controller 118 running the dedicated software 119 is arranged to control elements of the photoablator 101 such as the photoablator laser 102 and the beam positioner 104 as well as to store data.

The computer controller 118 running the dedicated software 119 is further arranged to fulfill tasks such as: control of the laser treatment, including definition of treatment area by computing data from the incorporated area detection means 113, control of laser beam positioner 104, control of the drilling feed-back unit 112, control of laser energy and frequency and other laser parameters important for accurate and safe laser procedure. Furthermore, the computer controller 118 comprises a data transfer interface, which allows transferring device, treatment and user data to the manufacturer in order arrange required maintenance, customer service. Such data transfer could occur on a regular basis e.g. once a month or every six months. For such data transfer an interface using electrical cables and a data net as the internet can be used. Optionally also wireless data transfer technology may be applied such as e.g. wireless communication standard protocols as UMTS, GPRS, etc..

The computer controller 118 comprises an interface to identify treatment specific identification information unique to every single treatment. This allows preventing the use of a docking module 110 in different patients and avoids cross-contamination of different diseases in different patients. Such treatment specific information might be incorporated as a bar code or in a chip on the disposable element either the docking module 110 or the docking unit 111 which is read before start of every treatment. In case the same bar code or information on the chip is read for different patients, the computer controller 118 inhibits the laser treatment. Therefore, the interface to read such information can be realized as a barcode reader or a chip connected to the docking module 110 when the docking unit 111 is attached to the treatment module 114.

The invention also covers all further features shown in the figures individually although they may not have been described in the afore or following description.

Also, other alternative embodiments of the photoablator according to the invention are conceivable. Explicitly mentioned in this context are:
- The photobalator can comprise a drilling feed-back unit without additionally having a treatment area definition unit.
- The photobalator can comprise a debris capture without additionally having a treatment area definition unit.

While the invention has been described with particular reference to certain embodiments thereof, it will be understood that changes and modifications may be made by those of ordinary skill within the scope and spirit of the following claims. In particular, the present invention covers further embodiments with any combination of features from different embodiments described above and below.

## Claims

1. A photoablator (1; 101) for perforating a nail, having a laser source (2; 102), optics (3; 103) for focusing a laser beam emitted by the laser source (2; 102) in a target plane, and beam positioning means (4; 104) for positioning the laser beam in relation to the nail, **characterized in that** the photoablator (1; 101) comprises a treatment area definition unit (13; 113) for automatically defining an area of the nail to perforate by means of the laser beam.

2. The photoablator (1; 101) according to claim 1, wherein the treatment area definition unit (13; 113) comprises area detection means.

3. The photoablator (1; 101) according to claim 2, wherein the area detection means comprise an optical sensor.

4. The photoablator (1; 101) according to claim 2, wherein the area detection means comprise an acoustical sensor.

5. The photoablator (1; 101) according to any one of the preceding claims, comprising a drilling feed-back unit (12; 112) with means for detecting a property of the tissue being perforated by the laser beam of the photoablator (1; 101) wherein the drilling feed-back unit (12; 112) stops the laser beam when the tissue property has a predefined value.

6. The photoablator (1, 101) according to claim 5, wherein the drilling feed-back unit (12; 112) comprises an acoustical sensor.

7. The photoablator (1, 101) according to claim 6, wherein the acoustical sensor comprises sound evaluating means.

8. The photoablator (1, 101) according to claim 5, wherein the drilling feed-back unit (12; 112) comprises an optical sensor.

9. The photoablator (1, 101) according to claim 8, wherein the optical sensor comprises color evaluating means.

10. The photoablator (1; 101) according to any one of the preceding claims, comprising a debris capture (5) with a pump (108), a coarse filter (106), and a fine filter (107), for evacuating debris from the nail.

11. The photoablator (1; 101) according to any one of the claims 1 to 9, comprising a debris capture (5) with adsorption means for adsorbing debris to evacuate the debris from the nail.

12. The photoablator (1; 101) according to any one of the preceding claims, comprising a docking unit (111) for positioning the nail in a predefined position.

13. The photoablator (1; 101) according to claim 12 having a treatment module (14; 114) comprising the laser source (2; 102), the optics (3; 103), the beam positioning means (4; 104), and a gripping portion and a docking module (10; 110) comprising the docking unit (111), wherein the treatment module (14; 114) is arranged for being manually dislocatable by means of the gripping portion and wherein the docking module (10; 110) is arranged to accommodate the treatment module (14; 114) while the nail is fixed in the predefined position.
